# EUROPEAN PATENT SPECIFICATION

(11) **EP 3 081 206 B1**
(45) Date of publication and mention of the grant of the patent: **19.02.2020**
(21) Application number: 16165370.4
(22) Date of filing: 14.04.2016
(51) Int. Cl.: A61K 8/25, A61K 8/29, A61K 8/49, A61Q 17/04

(54) **SUNSCREEN COMPOSITION**
SONNENSCHUTZZUSAMMENSETZUNG
COMPOSITION D'ECRAN SOLAIRE

(30) Priority: 15.04.2015 US 201562147676 P
(43) Date of publication of application: 19.10.2016
(73) Proprietor: Johnson & Johnson Consumer Inc., Skillman, NJ 08558 (US)
(72) Inventor: BLACHECHEN, Tatiana, 12241-070 Sao Jose dos Campos, SP (BR); DANTAS, Paula S. O., 12241-070 Sao Jose dos Campos, SP (BR); LORENZETTI, Danielle, 12241-070 Sao Jose dos Campos, SP (BR); NOGUEIRA, Luciano M., 12241-070 Sao Jose dos Campos, SP (BR); ZANATTA, Cinthia, Skillman, NJ New Jersey 08558 (US)
(74) Representative: Carpmaels & Ransford LLP

(56) References cited:
- EP-A1- 2 687 586
- WO-A1-2006/087066
- Verão Biovital: "Projeto Solar", , 2013, XP055281914, Retrieved from the Internet: URL:http://www.biovital.ind.br/2014/images /stories/downloads/projetos-e-protocolos/p rojeto-solar.pdf [retrieved on 2016-06-20]

## Description

### FIELD OF THE INVENTION

The present invention relates to sunscreen compositions suitable for topical application to the skin, which compositions prevent skin damage induced by ultraviolet (UV) and visible light radiation. In particular, the present invention relates to sunscreen composition comprising UV-absorbing agents and visible light absorbing agents.

### BACKGROUND OF THE INVENTION

The prolonged exposure to ultraviolet (UV) and visible light radiation, such as from the sun, can lead to the formation of light dermatoses and erythemas, as well as increase the risk of skin cancers, such as melanoma, and accelerate skin aging, such as loss of skin elasticity and wrinkling.

More specifically, visible radiation, commonly known as visible light, is the portion of the Solar electromagnetic spectrum range between 400 to 760nm and represents around 40% of the total solar energy that reaches the earth surface (UV is around 3-7%). Its main influence on human lives is the stimulation of human retina and distinction of objects colors, ranging from red to violet. Studies based on biological and cells cultivation responses demonstrate that skin damage associated with visible light, similar to UV damages, induces hyperpigmentation due to cumulative effects to chromophores, as well as Reactive Oxygen Species formation, which may lead to DNA damage and metalloproteinases expressions which may lead to collagen fiber breakdown and the inhibition of new fibers synthesis.

Numerous topical sunscreen compositions are commercially available with varying ability to protect the body from the harmful effects of ultraviolet and visible radiation. However, such sunscreen formulations are either ineffective in protecting against visible light, or are tinted by using relatively higher concentration of pigment. Challenges still exist to provide non-tinted sunscreen compositions that provide effective broader protection against both UV and visible radiation, without tinting the skin.

The present invention provides new sunscreen compositions employing the use of UV and visible radiation absorbing agents that are effective to protect both ultraviolet (UV) and visible radiation, without tinting the skin.

### SUMMARY OF THE INVENTION

Compositions of the present invention are sunscreen compositions comprising a primary UV-absorbing agent in an amount effective to absorb ultraviolet (UV) light, wherein the primary UV-absorbing agent is disodium phenyldibenzimidazole tetrasulfonate, and wherein the concentration of the primary UV-absorbing agent is 1.0% by weight of the composition or more; and 0.5 to 1% by weight of the composition of a visible light absorbing agent, wherein said visible light absorbing agent comprises inorganic pigment particles, and wherein the visible light absorbing agent comprises a silica-containing coating.

### DETAILED DESCRIPTION OF THE INVENTION

Unless defined otherwise, all technical and scientific terms used herein have the same meaning as commonly understood by one of ordinary skill in the art to which the invention belongs. As used herein, unless otherwise indicated, all hydrocarbon (e.g., alkyl, alkenyl) groups may be straight or branched chain groups. As used herein, unless otherwise indicated, the term "molecular weight" refers to weight average molecular weight, (Mw).

Unless defined otherwise, all concentrations refer to concentrations by weight of the composition. Also, unless specifically defined otherwise, the term "essentially free of," with respect to a class of ingredients, refers to the particular ingredient(s) being present in a concentration less than is necessary for the particularly ingredient to be effective to provide the benefit or property for which it otherwise would be used, for example, about 1% or less, or about 0.5% or less.

As used herein, "UV-absorbing" refers to a material or compound, e.g. a polymeric or non-polymeric, organic or inorganic, sunscreen agent or a chemical moiety, which absorbs radiation in some portion of the ultraviolet spectrum (290nm-400nm), such as one having an extinction coefficient of at least about 1000 mol⁻¹ cm⁻¹, for at least one wavelength within the above-defined ultraviolet spectrum, and provides further protection from UV light radiation via reflection and/or refraction.

As used herein, "visible-light absorbing" refers to a material or compound which absorbs radiation in some portion of the visible range (400nm-760nm), such as one having an extinction coefficient of at least about 1000 mol-1 cm-1, for at least one wavelength within the above-defined visible light spectrum, and provides further protection from the visible light radiation via absorption, reflection and/or refraction. Degrees of visible-light protection values disclosed and claimed herein are determined using the in-vitro method described herein below.

### PRIMARY UV-ABSORBING AGENTS

The invention relates to compositions comprising a primary UV-absorbing agent. Primary UV-absorbing agents include, but are not limited to, ethylhexyl triazone, 2-phenylbenzimidazole-5-sulfonic acid, bis-ethylhexyloxyphenylmethoxyphenyl triazine, disodium phenyldibenzimidazole tetrasulfonate, methylene bis-benzotriazolyl tetramethylbutylphenol, diethylamino hydroxy benzoyl hexyl benzoate and titanium dioxide and/or zinc oxide to which a silica coating has not been applied. According to the invention, the primary UV-absorbing agent is disodium phenyldibenzimidazole tetrasulfonate of the formula (I): 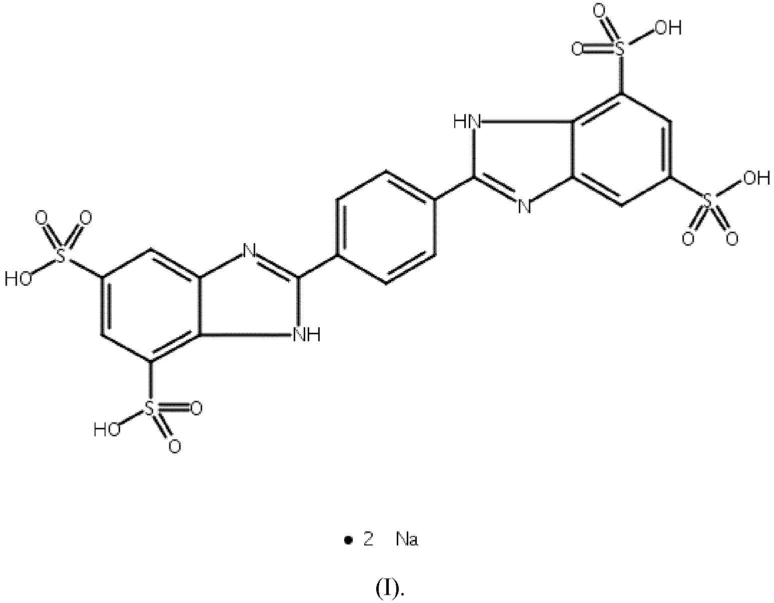 The primary UV-absorbing agent includes UV-absorbing moieties, as discussed herein below, and therefore absorbs radiation in some portion of the ultraviolet spectrum (290nm-400nm), such as one having an extinction coefficient of about 1000 mol⁻¹ cm⁻¹ or more, for example greater than 10,000 or 100,000 or 1,000,000 mol⁻¹ cm⁻¹, for at least one wavelength within the above-defined ultraviolet spectrum. The primary UV absorbing moiety may absorb predominantly in the UVA portion (320nm to 400 nm) or predominantly in the UV-B portion (290nm to 320 nm) of the ultraviolet spectrum. One such UV-absorbing moiety is a UV-absorbing triazole. By "UV-absorbing triazole" it is meant a UV-absorbing moiety containing a five-membered heterocyclic ring with two carbon and three nitrogen atoms. Typical UV-absorbing triazoles are benzotriazoles, which include the mentioned five-membered heterocyclic ring fused with a six-membered homocyclic aromatic ring. Examples of UV-absorbing agent include, for example, disodium phenyldibenzimidazole.

### VISBLE LIGHT-ABSORBING AGENTS

Compositions of the present invention comprise a visible light-absorbing agent. Such visible light-absorbing agents comprise inorganic pigment particles, such as certain inorganic oxides, including titanium dioxide, zinc oxide, and certain other transition metal oxides. According to certain embodiment of the invention, the inorganic pigment particles are titanium dioxide and zinc oxide, preferably titanium dioxide. The visible light-absorbing agent absorbs radiation in some portion of the visible light spectrum (400nm-760nm). Such visible light absorbing agents are typically solid particles having a diameter from about 1 nm to about 200 nm, preferably from about 5 to 120 nm.

Visible light-absorbing agents used in compositions of the present invention are coated with a silica film. As shown herein below, it was discovered that visible light-absorbing particles coated with silica provided compositions statistically significantly better in reflection of visible light than those compositions prepared with particles that were coated with alumina simethicone. Therefore, the silica films coated on the surface of the particles are essentially free of alumina, or are free of alumina. The thickness of the silica film deposited on the pigment particles may be from about 0.1 to about 100nm, preferably from 0.5 to 25nm. The silica-film-coated pigment particle may be formed by immersing the particle, e.g., titanium dioxide, in an aqueous, silica film-forming composition to deposit silica on the surface of titanium dioxide by precipitation of the silica on the titanium dioxide particles. The silica film has good compatibility with the substrate titanium dioxide and exhibits effective covering property. One example of such silica-coated titanium dioxide, comprising a pure inorganic film of silica (approx. 17% Silica), is available as Eusolex T-AVO from Merck KGaA in Germany.

### TOPICAL COMPOSITION

In one embodiment, a composition suitable for topical/cosmetic use for application to the human body (e.g., keratinaceous surfaces such as the skin, hair, lips, or nails), especially the skin, is provided. The composition includes one or more primary UV-absorbing agent and visible light-absorbing inorganic pigment particles, described herein. The concentration of the primary UV-absorbing agent is sufficient to provide an SPF of about 10 or greater, in the absence of secondary UV-absorbing agents, as described herein below. The concentration of the disodium phenyldibenzimidazole tetrasulfonate is 1.0% or more.

The concentration of silica-coated visible light-absorbing inorganic pigment particle contained in sunscreen compositions of the present invention must be present at levels effective to provide effective absorbance of visible light, yet not at levels commonly used to provide "tinting" of a composition, e.g., as in a tinted cream or paint. Therefore, the concentration is from 0.5% to 1%.

Compositions of the present invention may further comprise a secondary UV-absorbing agent. For example, the compositions of the invention will contain about 1% or more, or about 1.5% or more, of such UV-absorbing sunscreen agents. Examples of such compounds, sometimes referred to as "monomeric, organic UV-absorbers" include, but are not limited to, methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; butyl methoxydibenzoyl methane; glyceryl para-aminobenzoic acid (PABA); digalloyl trioleate; 3,(4-5 methylbenzylidene) camphor; methyl anthranilate; benzophenone-3; benzophenone-4; benzophenone-8; cinoxate; octocrylene; ethylhexyl dimethyl para-aminobenzoic acid, drometrizole trisiloxane; diethylhexyl butamido triazone; and polysilicone-15.

The compositions of the present invention may be used for a variety of cosmetic uses, especially for protection of the skin from UV radiation and visible light radiation. The compositions, thus, may be made into a wide variety of delivery forms. These forms include, but are not limited to, suspensions, dispersions, solutions, or coatings on water soluble or water-insoluble substrates (e.g., substrates such as organic or inorganic powders, fibers, or films). Suitable product forms include lotions, creams, gels, sticks, sprays, ointments, mousses, and compacts/powders. The composition may be employed for various end-uses, such as recreation or daily-use sunscreens, moisturizers, cosmetics/make-up, cleansers/toners, anti-aging products, or combinations thereof. The compositions of the present invention may be prepared using methodology that is well known by an artisan of ordinary skill in the field of cosmetics formulation.

Compositions of the present invention include a continuous water phase in which a discontinuous oil phase is substantially homogeneously distributed. The UV-absorbing agent and/or the visible light absorbing agent may be contained within the water or oil phase. In certain embodiments, the UV-absorbing agent is dissolved, as opposed to being dispersed or suspended, within the oil phase. The oil phase may, in turn, be stabilized within the water phase. The oil phase may be such that it is present in discrete droplets or units having an average diameter of about one micron to about 1000 microns, such as from about 1 micron to about 100 microns.

### TOPICAL CARRIER

The one or more Primary UV-absorbing agent and visible light absorbing inorganic pigment particle in the composition is combined with a "cosmetically-acceptable topical carrier," i.e., a carrier for topical use that is capable of having the other ingredients dispersed or dissolved therein, and possessing acceptable properties rendering it safe to use topically. As such, the composition may further include any of various functional ingredients known in the field of cosmetic chemistry, for example, emollients (including oils and waxes) as well as other ingredients commonly used in personal care compositions, such as humectants, thickeners, opacifiers, fragrances, dyes, solvents for the UV-absorbing polyester, among other functional ingredients. Suitable examples of solvents for the UV-absorbing polyester include dicaprylyl carbonate available as CETIOL CC from Cognis Corporation of Ambler, Pennsylvania.

In order to provide pleasant aesthetics, in certain embodiments of the invention, the composition is essentially free of volatile solvents; in particular, C₁-C₄ alcohols such as ethanol and isopropanol. Furthermore, the composition may be essentially free of ingredients that would render the composition unsuitable for topical use. As such, the composition may be essentially free of solvents such as volatile solvents, and, in particular, free of volatile organic solvents such as ketones, xylene, toluene, and the like.

### FILM-FORMING POLYMER

Compositions of the present invention may include a film forming polymer. The film-forming polymer may, when dissolved, emulsified, or dispersed in one or more diluents, permit a continuous or semi-continuous film to be formed when it is spread with a liquid vehicle onto a smooth substrate such as glass, and the liquid vehicle is allowed to evaporate. As such, the polymer may dry on the glass in a manner in which, over the area onto which it is spread, the film is predominantly continuous, rather than having a plurality of discrete, island-like structures. The films formed by applying compositions on the skin according to embodiments of the invention described herein, may be less than, on average, about 100 microns in thickness, such as less than about 50 microns.

In contrast to polymeric UV-absorbing agent and visible light absorbing inorganic pigment particle, film-forming polymers generally do not absorb appreciable ultraviolet or visible light radiation. However, by improving film formation, film-forming polymers may enhance the UV- and visible light protection (UV-A, UV-B or both) of the composition and/or enhance the waterproofing or water resistance of the composition.

The film-forming polymer may be an alkylated polyvinylpyrrolidone, such as a copolymer of vinylpyrrolidone and an α-olefin, such as a copolymer of vinylpyrrolidone and a long-chain (e.g., C₁₆ to C₃₀) α-olefin, e.g., GANEX V220, GANEX V216, GANEX WP660). In one particularly notable embodiment, the film-forming polymer is formed from 20% vinyl pyrrolidone and 80% C₁₆ olefin (1-hexadecene), such as GANEX V216. GANEX film-forming polymers are commercially available from ISP Specialty Chemicals (now Ashland Specialty Ingredients) of Wayne, New Jersey.

Compositions of the present invention may include additional film-forming polymers, including natural polymers such as polysaccharides or proteins and synthetic polymers such as polyesters, polacrylics, polyurethanes, vinyl polymers, polysulfonates, polyureas, polyoxazolines, and the like. Specific examples of additional film-forming polymers include, for example, hydrogenated dimer dilinoleyl/dimethylcarbonate copolymer, available from Cognis Corporation of Ambler, Pennsylvania as COSMEDIA DC; and water-dispersible polyesters, including sulfopolyesters such those commercially available from Eastman Chemical as EASTMAN AQ 38S.

The amount of film-forming polymer present in the composition may be from about 0.1% to about 5%, or from about 0.3% to about 3%, or from about 1% to about 2.5%.

### OIL-IN WATER EMULSIFIER

Compositions of the present invention may include one or more oil-in-water (O/W) emulsifiers selected from a group consisting of anionic emulsifiers and non-ionic emulsifiers. By "emulsifier," it is meant any of a variety of molecules that are suitable for emulsifying discrete oil-phase droplets in a continuous water phase. By "low molecular weight emulsifiers," it is meant emulsifiers having a molecular weight of about 2000 daltons or less, such as about 1000 daltons or less. The O/W emulsifier may be capable of lowering the surface tension of pure deionized water to 45 dynes per centimeter when added to pure deionized water at a concentration of O/W emulsifier of 0.5% or less at room temperature. The O/W emulsifier may have a hydrophile-lipophile balance (HLB) that is about 8 or more, such as about 10 or more.

In certain embodiments, the composition includes one or more anionic emulsifiers. Examples of suitable chemical classes of anionic emulsifiers are alkyl, aryl or alkylaryl, or acyl-modified versions of the following moieties: sulfates, ether sulfates, monoglyceryl ether sulfates, sulfonates, sulfosuccinates, ether sulfosuccinates, sulfosuccinamates, amidosulfosuccinates, carboxylates, amidoethercarboxylates, succinates, sarcosinates, amino acids, taurates, sulfoacetates, and phosphates. Notable anionic emulsifiers are salts of esters of phosphoric acid and cetyl alcohol, such as potassium salts of mixtures of esters of phosphoric acid and cetyl alcohol (e.g., 1-hexadecanol, dihydrogen phosphate, monopotassium salt). One notable example is potassium cetyl phosphate, hydrogenated palm glycerides, available as EMULSIPHOS from Symrise of Holzminden, Germany.

In certain embodiments, the concentration of the one or more anionic emulsifiers is from about 0.5% to about 6%, such as from about 1% to about 4%, such as from about 1% to about 2.5%.

In another embodiment of the invention, the composition includes one or more non-ionic emulsifiers. Examples of non-ionic emulsifiers include fatty amides, monoglycerides; sorbitan esters; polyoxyethylene derivatives of polyol esters; alkyl glucosides or polyglucosides; polyglyceryl esters; noncrosslinked silicone copolymers such as alkoxy or alkyl dimethicone copolyols, silicones having pendant hydrophilic moieties such as linear silicones having pendant polyether groups or polyglycerin groups; crosslinked elastomeric solid organopolysiloxanes comprising at least one hydrophilic moieties: polyethylene glycol, polypropylene glycol or polyglyceryl esters. According to one embodiment the non-ionic emulsifier has no alcohol functional groups. According to one embodiment of the invention, the non-ionic emulsifier has a molecular weight of about 10,000 daltons or less, such as about 7000 daltons or less.

According to one embodiment, the non-ionic surfactant is an ester of a fatty acid, such as various saturated or unsaturated, linear or branched, C₇-C₂₂ unethoxylated, aliphatic acids. The fatty acid may have from 14 to about 22 carbon atoms, such as from about 16 to about 18 carbon atoms. According to one embodiment, the non-ionic emulsifier is a polyether, such as selected from a fatty acid ester of glycerol (such as glyceryl stearate), a polyethylene glycol fatty acid ester (such as PEG-100 Stearate), and combinations thereof.

Specifically excluded from non-ionic surfactants are oil-gelling polymers, such as polymers that are capable of forming a gel with mineral oil at 25° C, such as when the oil-gelling polymer is mixed with mineral oil to a concentration of oil-gelling polymer that is between about of 0.25% to 2.0% by weight, the resulting mixture having a yield stress of about 5 pascals (Pa) or more, such as about 10Pa or more, such as from about 10Pa to about 1100 Pa. Examples of oil-gelling polymers are C₂-C₄ alkylcellulose polymers, such as ethylcellulose, which is an ethyl ether of cellulose comprising a long-chain polymer consisting of anhydroglucose units joined together by acetal linkages. Other examples of oil-gelling polymers are dibutyl ethylheaxanoyl glutamide and dibutyl lauroyl glutamide.

The concentration of non-ionic emulsifer may also range from about 1% to about 10%, such as from about 2% to about 6%, such as from about 2% to about 4%.

In certain embodiments, in addition to the emulsifier(s) discussed above, the composition includes an additional emulsifier such as one or more of an amphoteric emulsifier, a cationic emulsifier, and/or a polymeric emulsifier. Examples of suitable chemical classes of amphoteric emulsifier include alkyl betaines, amidoalkyl betaines, alkylamphoacetates; amidoalkyl sultaines; amphophosphates; phosphorylated imidazolines; carboxyalkyl alkyl polyamines; alkylimino-dipropionates; alkylamphoglycinates (mono or di); alkylamphoproprionates; N-alkyl β-aminoproprionic acids; and alkylpolyamino carboxylates. Examples of suitable chemical classes of cationic emulsifier include alkyl quaternaries, benzyl quaternaries, ester quaternaries, ethoxylated quaternaries, and alkyl amines. Examples of suitable chemical classes of polymeric emulsifiers include copolymers based on acrylamidoalkyl sulfonic acid such as Aristoflex® AVC and Aristoflex® HMB by Clariant Corporation; and Granthix APP by Grant Industries, Inc.

In certain embodiments, the composition includes an emollient used for the prevention or relief of dryness and for the protection of the skin, as well as solubilizing the UV-absorbing polyester. Suitable emollients include mineral oils, petrolatum, vegetable oils (e.g. triglycerides such as caprylic/capric triglyceride), waxes and other mixtures of fatty esters, including but not limited to esters of glycerol (e.g, isopropyl palmitate, isopropyl myristate), and silicone oils such as dimethicone. In certain embodiments, mixtures of triglycerides (e.g. caprylic/capric triclycerides) and esters of glycols (e.g. isopropyl myristate) may be used to solubilize the UV-absorbing agent and visible light absorbing inorganic pigment particle.

In certain embodiments, the composition includes a pigment suitable for providing color or hiding power. The pigment may be one suitable for use in a color cosmetic product, including compositions for application to the hair, nails and/or skin, especially the face. Color cosmetic compositions include, but are not limited to, foundations, concealers, primers, blush, mascara, eyeshadow, eyeliner, lipstick, nail polish and tinted moisturizers.

The pigment suitable for providing color or hiding power may be composed of iron oxides, including red and yellow iron oxides, titanium dioxide, ultramarine and chromium or chromium hydroxide colors, and mixtures thereof. The pigment may be a lake pigment, e.g., an organic dye such as azo, indigoid, triphenylmethane, anthraquinone, and xanthine dyes that are designated as D&C and FD&C blues, browns, greens, oranges, reds, yellows, etc., precipitated onto inert binders such as insoluble salts. Examples of lake pigments include Red #6, Red #7, Yellow #5 and Blue #1. The pigment may be an interference pigment. Examples of interference pigments include those containing mica substrates, bismuth oxycloride substrates, and silica substrates, for instance mica/bismuth oxychloride/iron oxide pigments commercially available as CHROMALITE pigments (BASF), titanium dioxide and/or iron oxides coated onto mica such as commercially available FLAMENCO pigments (BASF), mica/titanium dioxide/iron oxide pigments including commercially available KTZ pigments (Kobo products), CELLINI pearl pigments (BASF), and borosilicate-containing pigments such as REFLECKS pigments (BASF).

The compositions of the present invention may further comprise one or more other cosmetically active agent(s). A "cosmetically active agent" is a compound that has a cosmetic or therapeutic effect on the skin, e.g., agents to treat wrinkles, acne, or to lighten the skin. The cosmetically active agent will typically be present in the composition of the invention in an amount of from about 0.001 % to about 20% by weight of the composition, e.g., about 0.01% to about 10% such as about 0.1% to about 5% by weight of the composition.

In certain embodiments the composition has a pH that is from about 4.0 to about 8.0, such as from about 6.5 to about 7.7.

The compositions of the present invention may be prepared using mixing and blending methodology that is well known by an artisan of ordinary skill. In one embodiment of the invention, a method of making a composition of the present invention includes preparing an oil phase by mixing at least the UV-absorbing agent and visible light absorbing inorganic pigment particle with optional oil-soluble or oil-miscible ingredients; and preparing a water phase, by mixing water and optional water-soluble or water-miscible ingredients. The oil phase and the water phase may then be mixed in a manner sufficient to disperse the oil phase substantially homogeneously in the water phase such that the water phase is continuous and the oil phase discontinuous.

The compositions of the present invention can be used by topically administering to a mammal, e.g., by the direct laying on, wiping or spreading of the composition on the skin or hair of a human.

The following method was used in the evaluation of visible light protection effectiveness and in the following Examples.

### EXAMPLES

The following examples illustrate the preparation and efficacy of compositions of the present invention.

### Method of Evaluation of Visible Light Protection Effectiveness provided by cosmetic formulations

### Sample Preparation:

A blank PMMA (poly-methyl meta-acrylate) plate was prepared by spreading 0.2mL of glycerin or mineral oil on it until no residue was observed over the entire plate. The formulation was shaken well before weighing. A syringe was loaded with the formulation in an amount sufficient to result in a final concentration of 2mg/cm² when applied. The formulation was then dispensed over the expanse of the surface of a new PMMA plate as even droplets. The space between droplets was as even as possible. The droplets of formulation were then spread over the entire surface of a PMMA plate in 10 seconds to form a continuous film by moving the forefinger in small circular movements over the expanse of the PMMA plate surface. The plate was rotated at 90° and the finger was moved rightward and leftward during 5 seconds. After that, the plate again was rotated 90°. This two-step of rotating and moving the finger was repeated once in order to achieve 20 seconds of spreading and 4 90° rotations. Three replicates of each formulation were prepared.

### Equipment Settings:

For this method a multiport solar simulator equipped with a xenon arc lamp, manufactured by Solar Light Co, model 601 - lamp power of 300W, was used. The visible and UV light filters were removed from the solar simulator, leaving installed only the dichroic mirror; able to filter infra-red (IR) radiation. A spectroradiometer able to provide accurate spectral measurements over the 200-800nm wavelength range, manufactured by Optronic laboratories, model OL65A, equipped with an integrating sphere, was used to measure the solar radiation irradiance from the solar simulator. One of the six solar simulator light guides was connected to a sample compartment and this was connected to the integrating sphere. The other five light guides were closed. The desired wavelength range for the experiment, e.g., 250-800nm, was selected in the spectroradiometer, with increment of 1nm.

### Readings:

Each PMMA plate with glycerin was placed in the sample compartment and the spectra was collected over the desired radiation range, e.g., 400 to 760. The PMMA plate with sample was placed in the sample compartment and the spectra collected over the desired radiation range, e.g., 400 to 760nm. This step was repeated with all replicates. The area under the curve in the visible range (400-760nm) was determined for all evaluated samples, including the blank with glycerin. The level of visible light radiation filtered by the sample corresponds to the area mean values obtained for the sample divided by the area mean values obtained for the blank.

The following example illustrates the visible light absorption of certain compositions of the present invention. Inventive compositions (E1 and E2) include primary UV-absorbing agent and visible light absorbing inorganic pigment particle, and further include an alkylated polyvinylpyrrolidone, as well as an anionic or non-ionic emulsifier. These inventive examples and comparative examples were prepared by the process described below.

Water Phase: Add water, Disodium EDTA, Carbomer and Acrylates/C10-30 Alkyl Acrylate Crosspolymer. Start homogenization (4000 RPM - 6000 RPM) until total dispersion. Start mixing and add Sodium Hydroxide, under mixing add Phenylbenzimidazole Sulfonic Acid, Disodium Phenyl Dibenzimidazole Tetrasulfonate and Glycerin. Start heating batch until 82°C-85°C.

Oil Phase: In a separate vessel add Bis-Ethylhexyloxyphenol Methoxyphenyl Triazine, Butyl Methoxydibenzoylmethane, Octocrylene, Homosalate, Ethylhexyl Triazone, Tricontanyl PVP, Cetyl Palmitate, Caprylyl Methicone, Tocopheryl Acetate, Phenoxyethanol, Diisopropyl Adipate, Diisopropyl Sebacate, Disodium Cetearyl Sulfosuccinate, Cetearyl Alcohol; Cetearyl Glucoside, Dimethicone; Cetearyl Dimethicone Crosspolymer, Dimethicone, Isononyl Isononanoate. Start heating batch until 82°C-85°C.

Mix Oil and Water Phases and start cooling batch. Under mixing add Methylene Bis-Benzotriazolyl Tetramethylbutylphenol, Inulin Lauryl Carbamate and Hydroxyethyl Acrylate/Sodium Acryloyldimethyl Taurate Copolymer; Isohexadecane; Polysorbate 60.

Cool batch to 40°C and add Ethylhexylglycerin, Benzyl Alcohol, Extract Glycine Soya Seed, Capryloyl Glycine; Sarcosine; Cinnamomum Zeylanicum Extract, Titanium Dioxide; Silica, Aluminum Starch Octenylsuccinate, two types of Silicas and Nylon-12.

Pass through homogenizer for 3 minutes (4000 RPM - 6000 RPM).

**Table 1- Inventive Example/Formula (E1): Facial cream SPF 30**

| DESCRIPTION | SPF 30 (E1) |
|---|---|
| | |
| **VISIBLE LIGHT REDUCTION (%)** | 44 |
| **INCI name** | % |
| Water | 53.88 |
| Bis-Ethylhexyloxyphenol Methoxyphenyl Triazine | 1.50 |
| Butyl Methoxydibenzoylmethane | 1.50 |
| Octocrylene | 2.00 |
| Homosalate | 1.50 |
| Methylene Bis-Benzotriazolyl Tetramethylbutylphenol | 1.50 |
| Ethylhexyl Triazone | 1.50 |
| Chelating agents | 0.2 |
| Viscosity increase agent | 1.1 |
| Film former | 1.50 |
| Emollients | 8.2 |
| Anti-oxidant | 0.1 |
| Preservative system | 1.6 |
| Humectant | 2.0 |
| pH correction agent | 1.52 |
| Emulsifier agents | 4.6 |
| Absorbent agents | 8.0 |
| Extract | 2.8 |
| Phenylbenzimidazole Sulfonic Acid | 2.50 |
| Disodium Phenyl Dibenzimidazole Tetrasulfonate | 1.50 |
| Titanium Dioxide; Silica | 1.00 |

**Table 2 - Formula comparison between two non-tinted SPF 30 formulas, Invention Example 1(E1) and Comparative Example 1(C1)**

| Description | SPF 30 (E1) | SPF 30 (C1) |
|---|---|---|
| | | |
| **VISIBLE LIGHT REDUCTION (%)** | 44 | 14 |
| **INCI name** | % | % |
| Water | 53.88 | 72.55 |
| Bis-Ethylhexyloxyphenol Methoxyphenyl Triazine | 1.50 | 1.50 |
| Butyl Methoxydibenzoylmethane | 1.50 | 3.00 |
| Octocrylene | 2.00 | 3.00 |
| Homosalate | 1.50 | - |
| Methylene Bis-Benzotriazolyl Tetramethylbutylphenol | 1.50 | - |
| Ethylhexyl Triazone | 1.50 | 2.50 |
| Chelating agents | 0.2 | 0.2 |
| Viscosity increase agents | 1.1 | 0.4 |
| Film former | 1.5 | 0.5 |
| Emollients | 8.2 | 7.0 |
| Anti-oxidant agent | 0.1 | 0.1 |
| Preservative system | 1.6 | 1.5 |
| Humectant agents | 2.0 | 2.0 |
| Emulsifier agents | 4.6 | 1.5 |
| Absorbent agents | 8.0 | 2.0 |
| pH correction | 1.52 | 0.5 |
| extracts | 2.8 | - |
| Phenylbenzimidazole Sulfonic Acid | 2.50 | - |
| Disodium Phenyl Dibenzimidazole Tetrasulfonate | 1.50 | - |
| Titanium Dioxide; Silica | 1.00 | 0.50 |
| Parfum | - | 0.25 |

Invention Example 1 (E1) exhibits significant effectiveness regarding protection against visible light over Comparative example 1 (C1), i.e. 44% visible light reduction for E1 vs. against 14% visible light reduction for Comparative Example (C1).

**Table 3 Formula comparison between Comparative Example 2 (C2), Tinted and Inventive Example 1 (E1), non-tinted SPF 30 formulas**

| Description | Non tinted SPF 30 (E1) | Tinted SPF 30 (C2) |
|---|---|---|
| **VISIBLE LIGHT REDUCTION (%)** | 42.9 | 53.6 |
| | % | % |
| Absorbents agents | 8.0 | |
| Chelanting agents | 0.2 | |
| Viscosity increase agents | 1.1 | |
| pH adjust agent | 1.52 | |
| Humectant agents | 2.0 | |
| Emollients | 6.2 | |
| Phenylbenzimidazole Sulfonic Acid | 2.5000 | - |
| Disodium Phenyl Dibenzimidazole Tetrasulfonate | 1.5000 | - |
| Butyl Methoxydibenzoylmethane | 1.5000 | - |
| Octocrylene | 2.0000 | - |
| Homosalate | 1.5000 | - |
| Methylene Bis-Benzotriazolyl Tetramethylbutylphenol | 1.5000 | - |
| Water | 53.8800 | - |
| Film former | 1.5 | |
| Preservative system | 0.9 | |
| Emulsifier agents | 4.6 | |
| extracts | 2.8 | |
| Ethylhexyl Triazone | 1.5000 | 2.0000 |
| Tocopheryl Acetate | 0.1000 | 0.2500 |
| Phenoxyethanol | 0.7000 | 0.7000 |
| Dimethicone; Cetearyl Dimethicone Crosspolymer | 2.0000 | 18.0000 |
| Bis-Ethylhexyloxyphenol Methoxyphenyl Triazine | 1.5000 | 1.5000 |
| Diethylamino Hydroxybenzoyl Hexyl Benzoate | - | 5.0000 |
| Isododecane / Dimethicone/Bis-Isobutyl PPG-20 Crosspolymer | - | 48.4320 |
| C12-15 Alkyl Benzoate | - | 5.5000 |
| Ascorbic Acid | - | 5.0000 |
| Dimethicone/Vinyl Dimethicone Crosspolymer / Silica | - | 5.0000 |
| Ethylhexyl Methoxycinnamate | - | 5.0000 |
| Hydrogenated Polycyclopentadiene / Isododecane | - | 5.0000 |
| Mica | - | 2.5360 |
| Silica Dimethyl Silylate | - | 2.0000 |
| Butyrospermum Parkii Butter | - | 0.5000 |
| Phenylethyl Resorcinol | - | 0.4000 |
| Silica | - | 0.4000 |
| Titanium Dioxide; Silica | 1.0000 | - |
| Titanium Dioxide / Isononyl Isononanoate / Isopropyl Myristate / Stearalkonium Hectorite / Isopropyl Titanium Triisostearate / Propylene Carbonate / Polyhydroxystearic Acid | - | 18.0000 |
| CI 77492 / Isononyl Isononanoate / Isopropyl Myristate / Stearalkonium Hectorite / Polyhydroxystearic Acid / Isopropyl Titanium Triisostearate / Propylene Carbonate | - | 1.8000 |
| CI 77491 / Isononyl Isononanoate / Isopropyl Myristate / Stearalkonium Hectorite / Polyhydroxystearic Acid / Isopropyl Titanium Triisostearate / Propylene Carbonate | - | 0.3240 |
| CI 77499 / Isononyl Isononanoate / Isopropyl Myristate / Stearalkonium Hectorite / Polyhydroxystearic Acid / Isopropyl Titanium Triisostearate / Propylene Carbonate | - | 0.1080 |

Invention example 1 (E1) exhibits comparable effectiveness regarding protection against visible light over Comparative example 2 (C2), i.e. 42.9% visible light reduction for E1 vs. against 53.6% visible light reduction for Comparative Example (C2). Comparative example 2 (C2) is a tinted formulas, which is recognized to protect against visible light due the combination of high amount of titanium dioxide and inorganic pigments. Inventive example 1 (E1), despite being non-tinted, exhibits similar protection against visible light radiation. As shown in Table 3. Inventive Example 1 contains only 1% of titanium dioxide (coated with Silica), while the tinted formula Comparative 2 contains more than 20% of pigments compounds (comprehends around 12% of active pigment content). Inventive example 1 is shown to be effective in boosting the visible light protection.

**Table 4 - Formula Comparison between Inventive Example 2 (E2) and Comparative Example 3 (C3) and Comparative Example 4 (C4)**

| Description | Reference (SPF 30 formula Basis) (C3) | Reference + 1,5% DPDT (C4) | Reference + 1,5% DPDT + 1% Ti02/Silica (E2) |
|---|---|---|---|
| | | | |
| **VISIBLE LIGHT REDUCTION (%)** | 12 | 22 | 35 |

| **INCI name** | % | % | % |
|---|---|---|---|
| Water | **64.38** | **62.88** | **61.88** |
| Bis-Ethylhexyloxyphenol Methoxyphenyl Triazine | 1.50 | 1.50 | 1.50 |
| Butyl Methoxydibenzoylmethane | 1.50 | 1.50 | 1.50 |
| Octocrylene | 2.00 | 2.00 | 2.00 |
| Homosalate | 1.50 | 1.50 | 1.50 |
| Methylene Bis-Benzotriazolyl Tetramethylbutylphenol | 1.50 | 1.50 | 1.50 |
| Ethylhexyl Triazone | 1.50 | 1.50 | 1.50 |
| Chelating agents | 0.2 | 0.2 | 0.2 |
| Viscosity increase agent | 1.1 | 1.1 | 1.1 |
| Film former | 1.50 | 1.50 | 1.50 |
| Emollients | 8.2 | 8.2 | 8.2 |
| Anti-oxidant | 0.1 | 0.1 | 0.1 |
| Preservative system | 1.6 | 1.6 | 1.6 |
| Humectant | 2.0 | 2.0 | 2.0 |
| pH correction agent | 1.52 | 1.52 | 1.52 |
| Emulsifier agents | 4.6 | 4.6 | 4.6 |
| Absorbent agents | - | - | - |
| Extract | 2.8 | 2.8 | 2.8 |
| Phenylbenzimidazole Sulfonic Acid | 2.50 | 2.50 | 2.50 |
| Disodium Phenyl Dibenzimidazole Tetrasulfonate | - | 1.5 | 1.50 |
| Titanium Dioxide; Silica | - | - | 1.00 |

Inventive Example 2, which contains Titanium dioxide (coated with Silica) and the primary UVA filter Disodium Phenyl Dibenzimidazole Tetrasulfonate (DPDT), exhibits more significant improvement in visible light protection over Comparative Example 3, which contains neither Titanium dioxide (coated with Silica) nor the UVA filter Disodium Phenyl Dibenzimidazole Tetrasulfonate (DPDT), or Comparative Example 4, which contains only UVA filter Disodium Phenyl Dibenzimidazole Tetrasulfonate (DPDT).

**Table 5 Formula Comparison between Inventive Example 1 (E1) and Comparative Examples 5 (C5)**

| Description | SPF 30 (Original formula: TiO2/Silica) (E1) | SPF 30 (TiO2/Alumina,Simeth icone) (C5) |
|---|---|---|
| | | |
| **VISIBLE LIGHT REDUCTION (%)** | 44 | 38 |

| **INCI name** | % | % |
|---|---|---|
| Water | **53.88** | **53.88** |
| Bis-Ethylhexyloxyphenol Methoxyphenyl Triazine | 1.50 | 1.50 |
| Butyl Methoxydibenzoylmethane | **1.50** | **1.50** |
| Octocrylene | **2.00** | **2.00** |
| Homosalate | **1.50** | **1.50** |
| Methylene Bis-Benzotriazolyl Tetramethylbutylphenol | **1.50** | **1.50** |
| Ethylhexyl Triazone | **1.50** | **1.50** |
| Chelating agents | **0.2** | **0.2** |
| Viscosity increase agents | **1.1** | **1.1** |
| Film former | **1.5** | **1.5** |
| Emollients | **8.2** | **8.2** |
| Anti-oxidant agent | **0.1** | **0.1** |
| Preservative system | **1.6** | **1.6** |
| Humectant agents | **2.0** | **2.0** |
| Emulsifier agents | **4.6** | **4.6** |
| Absorbent agents | **8.0** | **8.0** |
| pH correction | **1.52** | **1.52** |
| extracts | **2.8** | **2.8** |
| Phenylbenzimidazole Sulfonic Acid | **2.50** | **2.50** |
| Disodium Phenyl Dibenzimidazole Tetrasulfonate | 1.50 | 1.50 |
| Titanium Dioxide; Silica | **1.00** | - |
| Titanium Dioxide; Alumina, Simethicone | - | **1.00** |

Inventive Example 1, which contains Titanium dioxide (coated with Silica) and the UVA filter Disodium Phenyl Dibenzimidazole Tetrasulfonate (DPDT), exhibits superior visible light protection over Comparative Example 5, which contains Titanium dioxide (coated with alumina simethicone).

**Table 6 Formula Comparison Summary between Inventive Examples (E1 and E2) and Comparative Examples (C1, C3, C4 and C5)**

| Description | SPF 30 (E1) | SPF 30 (C1) | TiO2/Alumina, Simethicone (C5) | Reference SPF 30 formula Basis) (C3) | Reference + 1% DPDT (C4) | Reference + 1% DPDT + 1% TiO2/Silica (E2) |
|---|---|---|---|---|---|---|
| **INCI name** | % | % | % | % | % | % |
| Water | 53.88 | 72.55 | 53.88 | 64.38 | 62.88 | 61.88 |
| Bis-Ethylhexyloxyphenol Methoxyphenyl Triazine | 1.50 | 1.50 | 1.50 | 1.50 | 1.50 | 1.50 |
| Butyl Methoxydibenzoylmethane | 1.50 | 3.00 | 1.50 | 1.50 | 1.50 | 1.50 |
| Octocrylene | 2.00 | 3.00 | 2.00 | 2.00 | 2.00 | 2.00 |
| Homosalate | 1.50 | - | 1.50 | 1.50 | 1.50 | 1.50 |
| Methylene Bis-Benzotriazolyl Tetramethylbutylphenol | 1.50 | - | 1.50 | 1.50 | 1.50 | 1.50 |
| Ethylhexyl Triazone | 1.50 | 2.50 | 1.50 | 1.50 | 1.50 | 1.50 |
| Chelating agents | 0.2 | 0.2 | 0.2 | 0.2 | 0.2 | 0.2 |
| Viscosity increase agents | 1.1 | 0.4 | 1.1 | 1.1 | 1.1 | 1.1 |
| Film former | 1.5 | 0.5 | 1.5 | 1.50 | 1.50 | 1.50 |
| Emollients | 8.2 | 7.0 | 8.2 | 8.2 | 8.2 | 8.2 |
| Anti-oxidant agent | 0.1 | 0.1 | 0.1 | 0.1 | 0.1 | 0.1 |
| Preservative system | 1.6 | 1.5 | 1.6 | 1.6 | 1.6 | 1.6 |
| Humectant agents | 2.0 | 2.0 | 2.0 | 2.0 | 2.0 | 2.0 |
| Emulsifier agents | 4.6 | 1.5 | 4.6 | 4.6 | 4.6 | 4.6 |
| Absorbent agents | 8.0 | 2.0 | 8.0 | 8.0 | 8.0 | 8.0 |
| pH correction | 1.52 | 0.5 | 1.52 | - | - | - |
| extracts | 2.8 | - | 2.8 | 2.8 | 2.8 | 2.8 |
| Phenylbenzimidazole Sulfonic Acid | 2.50 | - | 2.50 | 2.50 | 2.50 | 2.50 |
| Disodium Phenyl Dibenzimidazole Tetrasulfonate | 1.50 | - | 1.50 | - | 1.5 | 1.50 |
| Titanium Dioxide; Silica | 1.00 | 0.50 | - | - | - | 1.00 |
| Titanium Dioxide; Alumina, Simethicone | - | - | 1.00 | - | - | - |
| Parfum | - | 0.25 | - | - | - | - |

| | SPF 30 | SPF 30 | TiO2/Alumina, Simethicone | Reference (SPF 30 formula Basis) | Reference + 1,5% DPDT | Reference + 1,5% DPDT + 1% TiO2/Silica |
|---|---|---|---|---|---|---|
| **VISIBLE LIGHT REDUCTION (%)** | 44 | 14 | 38 | 12 | 22 | 35 |

The main key components identified as responsible for delivering boost effect on visible light protection are UV-absorbing agent, Disodium Phenyl Dibenzimidazole Tetrasulfonate, and Titanium Dioxide coated with Silica.

**Table 7 Concentration Ranges for Disodium Phenyl Dibenzimidazole Tetrasulfonate and Silica Coated Titanium Dioxide**

| Description | SPF 30 | | |
|---|---|---|---|
| | | | |
| **INCI** | **%** | **Minimum (%)** | **Maximum (%)** |
| Disodium Phenyl Dibenzimidazole | 1 | 0.5 | 5.0 |
| Titanium Dioxide; Silica | 1 | 0.2 | 25 |

**Table 8 Concentration Ranges for Key Ingredients in the Inventive Example**

| Description | SPF 30 | | |
|---|---|---|---|
| | | | |
| **INCI** | | **Minimum (%)** | **Maximum (%)** |
| Phenylbenzimidazole Sulfonic Acid | 2 | 0.5 | 8.0 |
| Bis-Ethylhexyloxyphenol Methoxyphenyl | 1 | 0.5 | 10 |
| Butyl Methoxydibenzoylmethane | 1 | 0.5 | 10 |
| Octocrylene | 2 | 0.5 | 12 |
| Methylene Bis-Benzotriazolyl | 1 | 0.5 | 10 |
| Ethylhexyl Triazone | 1 | 0.5 | 10 |
| Homosalate | 1 | 0.5 | 15 |
| Aluminum Starch Octenylsuccinate | 2 | 0.2 | 10 |
| Silica | 2 | 0.2 | 15 |
| Nylon-12 | 1 | 0.2 | 15 |

## Claims

1. A sunscreen composition, comprising:
a primary UV-absorbing agent in an amount effective to absorb ultraviolet light, wherein said primary UV-absorbing agent is disodium phenyldibenzimidazole tetrasulfonate, and wherein the concentration of said primary UV-absorbing agent is 1.0% by weight of the composition or more; and
0.5 to 1% by weight of the composition of a visible light absorbing agent, wherein said visible light absorbing agent comprises inorganic pigment particles, and wherein said visible light absorbing agent comprises a silica-containing coating.

2. The sunscreen composition of claim 1 wherein said visible light absorbing agent is selected from the group consisting of inorganic oxides and other transition metal oxides.

3. The sunscreen composition of claim 1 or claim 2 wherein said silica-containing coating is a film.

4. The sunscreen composition of claim 3 wherein said silica-containing film is essentially free of alumina.

5. The sunscreen composition of claim 3 or claim 4 wherein the thickness of said film on said visible light absorbing agent is from 0.1 to 100nm.

6. The sunscreen composition of any of the preceding claims wherein said visible light absorbing agent is titanium dioxide.

7. The sunscreen composition of any of the preceding claims wherein said silica-containing coating is continuous.

8. The sunscreen composition of any of the preceding claims further comprising a secondary UV-absorbing agent.

9. The sunscreen composition of claim 1, comprising:
1.5% by weight of disodium phenyldibenzimidazole tetrasulfonate;
1% by weight of the visible light absorbing agent, wherein the visible light absorbing agent is titanium dioxide;
2.5% by weight of phenylbenzimidazole sulfonic acid;
1.5% by weight of bis-ethylhexyloxyphenol methoxyphenyl triazine;
1.5% by weight of butyl methoxydibenzoylmethane;
2% by weight of octocrylene;
1.5% by weight of methylene bis-benzotriazolyl tetramethylbutylphenol;
1.5% by weight of ethylhexyl triazone; and
1.5% by weight of homosalate.

## Patentansprüche

1. Sonnenschutzzusammensetzung, umfassend:
ein primäres UV-absorbierendes Mittel in einer zur Absorption ultravioletten Lichts wirksamen Menge, wobei es sich bei dem primären UV-absorbierenden Mittel um Dinatriumphenyldibenzimidazoltetrasulfonat handelt und wobei die Konzentration des primären UV-absorbierenden Mittels 1,0 %, bezogen auf das Gewicht der Zusammensetzung, oder mehr beträgt, und
0,5 bis 1 %, bezogen auf das Gewicht der Zusammensetzung, eines Absorptionsmittels für sichtbares Licht, wobei das Absorptionsmittel für sichtbares Licht anorganische Pigmentpartikel umfasst und wobei das Absorptionsmittel für sichtbares Licht eine siliciumdioxidhaltige Beschichtung umfasst.

2. Sonnenschutzzusammensetzung nach Anspruch 1, wobei das Absorptionsmittel für sichtbares Licht aus der Gruppe bestehend aus anorganischen Oxiden und weiteren Oxiden von Übergangsmetallen ausgewählt ist.

3. Sonnenschutzzusammensetzung nach Anspruch 1 oder 2, wobei es sich bei der siliciumdioxidhaltigen Beschichtung um einen Film handelt.

4. Sonnenschutzzusammensetzung nach Anspruch 3, wobei die siliciumdioxidhaltige Beschichtung im Wesentlichen frei von Aluminiumoxid ist.

5. Sonnenschutzzusammensetzung nach Anspruch 3 oder 4, wobei die Dicke des Films auf dem Absorptionsmittel für sichtbares Licht 0,1 bis 100 nm beträgt.

6. Sonnenschutzzusammensetzung nach einem der vorhergehenden Ansprüche, wobei es sich bei dem Absorptionsmittel für sichtbares Licht um Titandioxid handelt.

7. Sonnenschutzzusammensetzung nach einem der vorhergehenden Ansprüche, wobei die siliciumdioxidhaltige Beschichtung durchgehend ist.

8. Sonnenschutzzusammensetzung nach einem der vorhergehenden Ansprüche, die ferner ein sekundäres UV-absorbierendes Mittel umfasst.

9. Sonnenschutzzusammensetzung nach Anspruch 1, umfassend:
1,5 Gewichts-% Dinatriumphenyldibenzimidazoltetrasulfonat,
1 Gewichts-% Absorptionsmittel für sichtbares Licht, wobei es sich bei dem Absorptionsmittel für sichtbares Licht um Titandioxid handelt,
2,5 Gewichts-% Phenylbenzimidazolsulfonsäure,
1,5 Gewichts-% Bisethylhexyloxyphenolmethoxyphenyltriazin,
1,5 Gewichts-% Butylmethoxydibenzoylmethan,
2 Gewichts-% Octocrylen,
1,5 Gewichts-% Methylenbisbenzotriazolyltetramethylbutylphenol,
1,5 Gewichts-% Ethylhexyltriazon und
1,5 Gewichts-% Homosalat.

## Revendications

1. Composition de protection solaire comprenant :
un agent absorbant les UV primaire en une quantité efficace pour absorber la lumière ultraviolette,
dans laquelle l'agent absorbant les UV primaire est le tétrasulfonate de phényldibenzimidazole disodique, et dans laquelle la concentration dudit agent absorbant les UV primaire est de 1,0 % en poids de la composition ou plus ; et
de 0,5 à 1 % en poids de la composition d'un agent absorbant la lumière visible,
dans laquelle ledit agent absorbant la lumière visible comprend des particules de pigment inorganiques, et
dans laquelle ledit agent absorbant la lumière visible comprend un revêtement contenant de la silice.

2. Composition de protection solaire selon la revendication 1 dans laquelle ledit agent absorbant la lumière visible est choisi dans le groupe constitué par les oxydes inorganiques et d'autres oxydes de métaux de transition.

3. Composition de protection solaire selon la revendication 1 ou la revendication 2 dans laquelle ledit revêtement contenant de la silice est un film.

4. Composition de protection solaire selon la revendication 3 dans laquelle ledit film contenant de la silice est essentiellement exempt d'alumine.

5. Composition de protection solaire selon la revendication 3 ou la revendication 4 dans laquelle l'épaisseur dudit film sur ledit agent absorbant la lumière visible est de 0,1 à 100 nm.

6. Composition de protection solaire selon l'une quelconque des revendications précédentes dans laquelle ledit agent absorbant la lumière visible est le dioxyde de titane.

7. Composition de protection solaire selon l'une quelconque des revendications précédentes dans laquelle ledit revêtement contenant de la silice est continu.

8. Composition de protection solaire selon l'une quelconque des revendications précédentes comprenant en outre un agent absorbant les UV secondaire.

9. Composition de protection solaire selon la revendication 1, comprenant :
1,5 % en poids de tétrasulfonate de phényldibenzimidazole disodique ;
1 % en poids de l'agent absorbant la lumière visible, l'agent absorbant la lumière visible étant le dioxyde de titane ;
2,5 % en poids d'acide phénylbenzimidazolesulfonique ;
1,5 % en poids de bis-éthylhexyloxyphénolméthoxyphényltriazine ;
1,5 % en poids de butylméthoxydibenzoylméthane ;
2 % en poids d'octocrylène ;
1,5 % en poids de méthylène-bis-benzotriazolyltétraméthylbutylphénol ;
1,5 % en poids d'éthylhexyltriazone ; et
1,5 % en poids d'homosalate.
